# EUROPEAN PATENT APPLICATION

(11) **EP 1 958 579 A1**
(43) Date of publication of application: **20.08.2008**
(21) Application number: 08002639.6
(22) Date of filing: 13.02.2008
(51) Int. Cl.: A61B 17/70, A61B 17/86, A61B 17/88

(54) **Vertebra connection member and nut driver**

(30) Priority: 14.02.2007 JP 2007033975
(71) Applicant: Showa Ika Kohgyo Co., Ltd., Toyohashi-shi Aichi 441-8026 (JP)
(72) Inventor: Laager, Charles-Marc, 20148 Milano (IT); Boriani, Stefano, 40121 Bologna (IT); Ali, Giuseppe, 40134 Bologna (IT); Oribe, Kazuya, Minato-ku Tokyo 105-0011 (JP)
(74) Representative: Behm, Sonja Marianne

(57) **Abstract**

A vertebra connection member (1) has: a screw member (3) that is screwed (10) in a vertebra; a connector member (4) that connects a rod (2) and the screw member; a first nut member (5) that fixes the screw member and connector member; a second nut member (6) that prevents the loosening of the first nut member; and a fixing member (7) that fixes the rod and connector member. The first and second nut members are formed to have a cylindrical shape; and engagement concave portions (26,28) of a concave curved shape are formed in the outer peripheral surface of the nut members from the rear ends (5a,6a) in the screw-in direction to the front ends (5b,6b) in the screw-in direction, in the direction in which the nut members are screwed onto the second male portion (11).

## Description

### BACKGROUND OF THE INVENTION

### Field of the Invention

The present invention relates to a vertebra connection member that connects a plurality of vertebrae via a rod, and to a nut driver.

### Related Background Art

Examples of known vertebra connection members that connect a plurality of vertebrae include a connection member described in a pamphlet "The ISOLA Spinal System" DS002P01 200503 published by ROBERT REID INC. and a connection member described in a pamphlet "Monarch SPINE SYSTEM" DS026P01 200505 published by ROBERT REID INC.

In these conventional vertebra connection members, screw members screwed into a plurality of vertebrae and a rod are connected by a connector member. For this purpose, a screw member insertion orifice for inserting the screw member and a rod insertion orifices for inserting the rod are formed in the connector member. A first male threaded portion for screwing into a vertebra is formed at one end of the screw member, a second male threaded portion is formed at the other end, and a locking portion that is larger in scale that the second male threaded portion is formed between the first male threaded portion and the second male threaded portion. The screw member and connector member are fixed by inserting the screw member into the connector member, screwing a hexagonal nut onto the second male threaded portion, and sandwiching the connector member between the nut and the locking portion.

Where a rod is then inserted and fixed in the rod insertion orifice, the screw member and the rod are connected and a plurality of vertebrae are connected.

### SUMMARY OF THE INVENTION

However, where a hexagonal nut is used for fixing the screw member and connector member, as in the conventional vertebra connection member, the thickness of the nut driver is locally decreased in the circumferential direction and becomes uneven. As a result, the thickness of the nut driver as a whole has to be increased. Therefore, a space for turning the nut driver has to be ensured when the hexagonal nut is screwed onto the second male thread portion of the screw member in the conventional vertebra connection member. The resultant problem is that the size of the connection member cannot be decreased.

Accordingly, it is an object of the present invention to provide a vertebra connection member that enables the size decrease of the connector member and to provide a nut driver.

The vertebra connection member in accordance with the present invention is a vertebra connection member that connects a plurality of vertebrae via a rod, comprising: a screw member, in which a first male threaded portion to be screwed into a vertebra is formed at one end, a second male threaded portion is formed on a side closer to the other end than the first male threaded portion, and a locking portion having a larger diameter than the second male threaded portion is formed between the first male threaded portion and the second male threaded portion; a connector member having formed therein a screw member insertion portion for inserting the screw member from a side closer to the second male threaded portion than the locking portion, thereby locking the locking portion, and a rod insertion portion into which the rod is inserted, the connector member connecting the screw member and the rod; and a substantially cylindrical nut member having formed on an inner peripheral surface thereof a female threaded portion that conforms in shape to the second male threaded portion and on an outer peripheral surface thereof one and more engagement concave portions of a concave curved shape oriented forward in a screw-in direction from a rear end in the screw-in direction of the female threaded portion.

In the vertebra connection member in accordance with the present invention, by forming a substantially cylindrical nut member and forming one and more engagement concave portions of a concave curved shape on the outer peripheral surface thereof, it is possible to engage with a nut driver by the engagement concave portions, without providing a portion that protrudes from the substantially cylindrical outer peripheral surface. As a result, the nut member holding portion of the nut driver is not thin partially and the thickness of the nut member holding portion can be thinned overall. Therefore, when the second male thread member is screwed into the nut member, a space necessary for rotating the nut driver can be decreased and the connector member can be reduced in size. Further, because the engagement concave portions of the nut member is formed to have a concave curved shape, the nut member can receive the rotation force applied from the nut driver by the concave curved surface thereof. Therefore, the surface area of contact with the nut driver can be enlarged and damage of nut member and nut driver can be inhibited.

In this case, the engagement concave portions are preferably formed to have a circular arc shape, the center thereof being in a point on a circumference forming the outer peripheral surface of the nut member. With such vertebra connection member, the frontmost end in the rotation direction of the nut driver that comes into contact with the engagement concave portions are on the inner side in the radial direction from the outer peripheral surface of the nut portion. As a result, the loss of rotation force received by the nut member from the nut driver can be reduced and the nut member can be reliably rotated.

Further, the engagement concave portions are preferably formed up to the front end in the screw-in direction. With such vertebra connection member, because the engagement concave portions can be formed from the front end in the screw-in direction of the nut member to the other end in the screw-in direction, the nut member can be easily machined.

The engagement concave portions are preferably formed, in the screw-in direction of the female threaded portion, at the front end up to a point where a predetermined distance begins. With such vertebra connection member, by providing a zone where no engagement concave portions are formed at the front end in the screw-in direction of the nut member, the nut driver can be prevented from getting forward through the nut member in the screw-in direction. Therefore, the nut drive can be prevented from erroneously rotating another nut member at the same time, and the screw-in position (or screw-in amount) of the nut member that is being presently screwed can be determined.

Further, it is preferred that the engagement concave portions be formed with equal spacing in the circumferential direction. With such vertebra connection member, the rotation forces inputted from the engagement concave portions can be balanced and, therefore, the nut member can be smoothly rotated.

The engagement concave portions are preferably formed in three locations. For example, where the engagement concave portions are formed in two locations or one location in the circumferential direction, the balance of forces applied from the engagement concave portions can be easily lost. As a result, the nut member is difficult to rotated smoothly. On the other hand, where the engagement concave portions are formed in four or more locations in the circumferential direction, the machining cost of the engagement concave portions is increased. By contrast, with the above-described vertebra connection member, because the engagement concave portions are formed in three locations in the circumferential direction, the forces inputted from the engagement concave portions can be easily balanced, and the nut member can be rotated smoothly, while inhibiting the machining cost.

The nut driver in accordance with the present invention has a nut member holding portion that covers the outer circumferential surface of the nut member, wherein the nut member holding portion has an engagement convex portion in the form of a convex curved surface that engages with the at least one engagement concave portions.

With the nut driver in accordance with the present invention, the nut member holding portion of the nut driver is not thin partially and the thickness of the nut member holding portion can be thinned overall. Therefore, when the second male thread member is screwed into the nut member, a space necessary for rotating the nut driver can be decreased and the connector member can be reduced in size. Further, because the engagement convex portion is formed to have a convex curved shape, the rotation force can be transmitted to the nut member by this convex curved surface. Therefore, the surface area of contact with the nut member can be enlarged and damage of nut member and nut driver can be inhibited.

Further, where the nut member holding portion has a shape that covers the entire circumference of the nut member, when the nut driver is rotated, the nut member holding portion can receive the force that acts outwardly in the radial direction upon the engagement convex portion due to the reaction force from the at least one engagement concave portions, the loss of force rotating the nut member can be reduced, and the nut member can be reliably rotated.

The present invention can be more thoroughly understood based on the detailed explanation and appended drawings presented below. These drawings are presented merely to illustrate the invention and should not be construed as placing any limitation thereon.

### BRIEF DESCRIPTION OF THE DRAWINGS

FIG. 1 is a perspective view illustrating the state in which three vertebra connection members of the embodiment of the present invention are connected;
FIG. 2 is a top view of the vertebra connection member;
FIG. 3 is a perspective view of the nut member;
FIG. 4 is a perspective view of the nut member;
FIG. 5 is a top view of the nut member;
FIG. 6 is a front view illustrating the driver of the embodiment of the present invention;
FIG. 7 is a perspective view illustrating, with partial enlargement, the lower portion of the nut driver;
FIG. 8 is a bottom view of the nut driver;

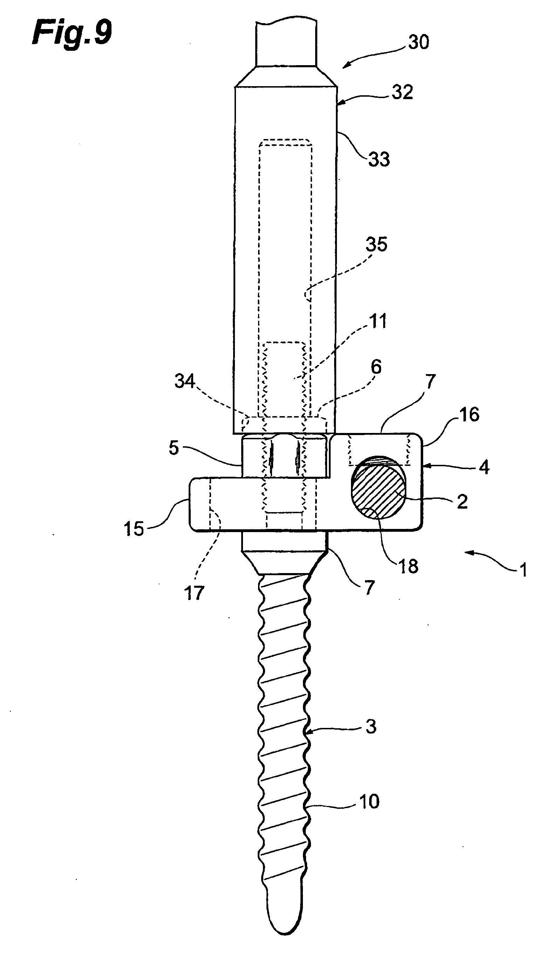

FIG. 9 illustrates a state in which the nut member is tightened by the nut driver; and
FIG. 10 is a perspective view illustrating another nut member.

### DESCRIPTION OF THE PREFERRED EMBODIMENTS

Embodiments of the present invention will be described below in greater detail with reference to the drawings. In the explanation of the drawings, identical elements will be assigned with identical reference symbols and redundant explanation thereof will be omitted.

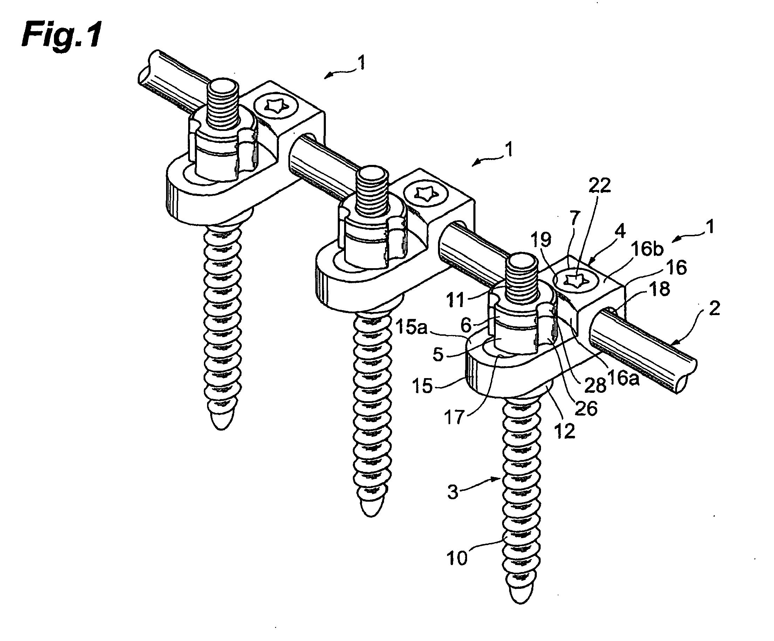

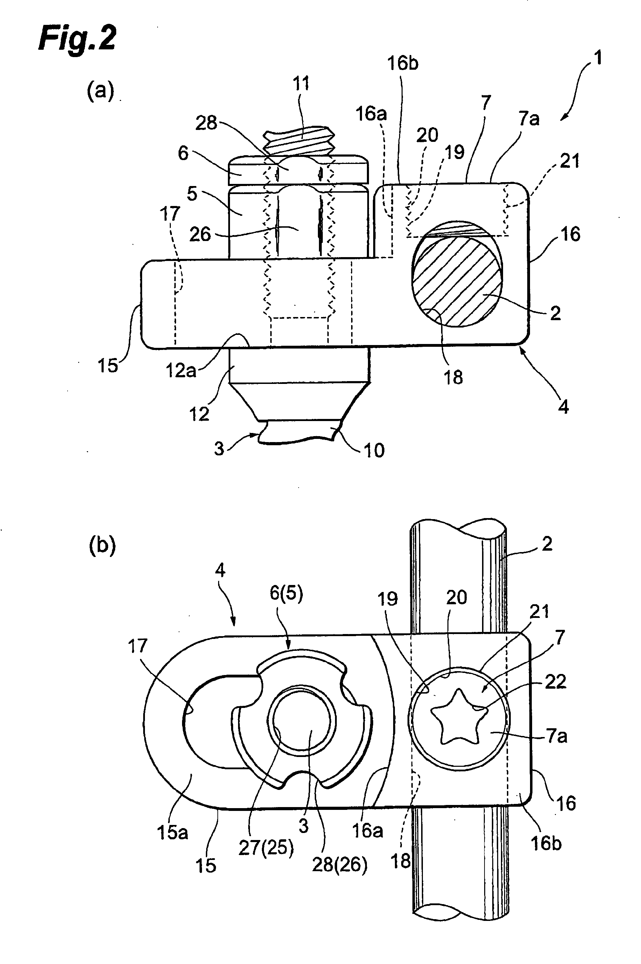

First, a vertebra connection member of an embodiment of the present invention will be explained. FIG. 1 is a perspective view illustrating the state in which three vertebra connection members of the embodiment of the present invention are connected. FIG. 2 is a top view of the vertebra connection member.

As shown in FIG. 1 and FIG. 2, vertebra connection members 1 of the present embodiment connect a plurality of vertebrae via a rod 2, each vertebra connection member comprising a screw member 3 that will be screwed into a vertebra, a connector member 4 that connects the rod 2 and the screw member 3, a nut member 5 that fixes the screw member 3 and the connector member 4, a nut member 6 that tightens the nut member 5, and a fixing member 7 that fixes the rod 2 and the connector member 4.

In order to connect a plurality of vertebra connection members 1, the rod 2 is formed to have an elongated cylindrical shape that enables the connection of a plurality of vertebra connection members 1.

The screw member 3 that will be screwed into a vertebra is formed to have a rod-like shape, and a first male threaded portion 10 that will be screwed into the vertebra is formed at one end of the screw member. A second male threaded portion 11 onto which the nut members 5, 6 are screwed is formed at the other end of the screw member 3. A locking portion 12 that is larger in diameter than the second male threaded portion 11 is formed between the first male threaded portion 10 and the second male threaded portion 11. An upper end surface 12a (upper end surface in (a) of FIG. 2) of the locking portion 12 is formed to have a flat shape so as to prevent the locking portion from playing when the upper end portion thereof is abutted against the connector member 4.

The connector member 4 that connects the rod 2 and the screw member 3 comprises a screw member fixing portion 15 for fixing the screw member 3 and a rod fixing portion 16 for fixing the rod 2. The screw member fixing portion 15 is formed to be thinner than the rod fixing portion 16, and a screw member insertion orifice 17 passing through the screw member fixing portion 15 is formed in the center of the screw member fixing portion so that the second male threaded portion 11 of the screw member 3 can be inserted therein.

The screw member insertion orifice 17 is formed to have an elliptical shape with the long axis direction being along the direction of withdrawing from the rod fixing member 16, so that the distance between the inserted screw member 3 and the rod fixing portion 16 can be adjusted. Further, the diameter of the screw member insertion orifice 17 is set to be larger than the maximum diameter of the second male threaded portion 11 and smaller than the maximum diameter of the locking portion 12, so that the locking portion 12 of the inserted screw member 3 be locked by the screw member fixing portion 15 and do not pass through the screw member insertion orifice 17.

Further, the rod fixing portion 16 is formed to be thicker than the screw member 3 and to rise above an upper surface 15a (upper surface in (a) of FIG. 2) of the screw member fixing portion 15. A side surface 16a (left side surface in (b) of FIG. 2) on the side of the screw member fixing portion 15 of the rod fixing member 16 is formed to have a concave curved shape that follows the shape of the nut members 5, 6.

A rod insertion orifice 18 that passes through in the direction different from that of the screw member insertion orifice 17, so that the rod 2 can be inserted in the direction different from that of the screw member insertion orifice 17, is formed in the central portion in the up-down diction (up-down direction in (a) of FIG. 2) of the rod fixing member 16. In the present embodiment, the screw member insertion orifice 17 and the rod insertion orifice 18 are formed through respective portions at an angle of 90 degrees to each other.

Further, in the rod fixing portion 16, there is formed an orifice 19 for a fixing member that passes through in the up-down direction (up-down direction in (a) of FIG. 2) from an upper surface 16b (upper surface in (a) of FIG. 2) of the rod fixing portion 16 to the rod insertion orifice 18 in order to fix the rod 2 inserted into the rod insertion orifice 18. A female treated portion 20 is formed on the inner peripheral surface of the orifice 19 for a fixing member, so that the fixing member 7 can be screwed therein.

The fixing member 7 that is screwed in the orifice 19 for a fixing member of the rod fixing portion 16 to fix the rod 2 is formed to have a cylindrical shape, and a male threaded portion 21 conforming to the female threaded portion 20 of the orifice 19 for a fixing member is formed in the outer peripheral surface of the fixing member. A star-shaped concave portion 22 for inserting a TORX (star-shaped) wrench that will rotate the fixing member 7 is formed in an upper surface 7a (upper surface in (a) of FIG. 2) of the fixing member 7.

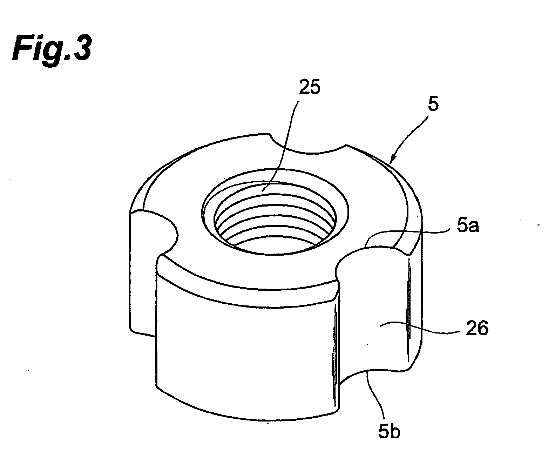

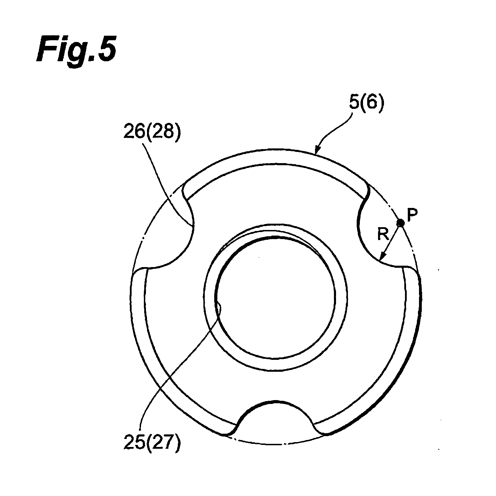

The nut member 5 that fixes the screw member 3 and connector member 4 is formed to have a cylindrical shape, as shown in FIG. 3 and FIG. 5. A female threaded portion 25 that conforms to the second female threaded portion 11 of the screw member 3 is formed in the inner peripheral surface of the nut member 5. The outer diameter of the nut member 5 is set larger than the diameter of the screw member insertion orifice 17 in the short radius direction, so that the nut member 5 is locked by the screw member fixing portion 15 and does not pass through the screw member insertion orifice 17. Further, an engagement concave portion 26 having a concave curved shape is formed in the outer peripheral surface of the nut member 5.

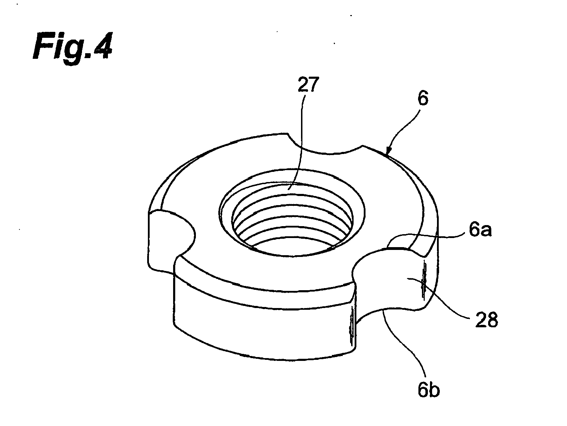

Further, the nut member 6 serving to tighten the nut member 5 is formed to be thinner than the nut member 5, as shown in FIG. 4 and FIG. 5, and to have a cylindrical shape similarly to the nut member 5. A female threaded portion 27 conforming to the second male threaded portion 11 of the screw member 3 is formed in the inner peripheral surface of the nut member 6. The outer diameter of the nut member 6 is set larger than the diameter of the screw member insertion orifice 17 in the short radius direction, so that the nut member 6 is locked by the screw member fixing portion 15 and does not pass through the screw member insertion orifice 17. As shown in FIG. 3 and FIG. 4, engagement concave portions 28 having a concave curved shape are formed in the outer peripheral surface of the nut member 6 from the rear end 5a, 6a (upper end in FIG. 3 and FIG. 4) in the screw-in direction to the front end 5b, 6b in the screw-in direction along the screw-in direction of the nut members 5, 6 with respect to the second male threaded portion 11.

As shown in FIG. 5, these engagement concave portions 26, 28 of the nut members 5, 6 formed to have a circular arc shape with the center in point P on a circumference forming the outer peripheral portion of the nut members 5, 6 in the transverse section of the nut members 5, 6 and are also formed in three locations with equal spacing (spacing of 120 degrees) in the circumferential direction of nut members 5, 6. The distance from the engagement concave portions 26, 28 to the inner peripheral surface is computed and the radius R of circular arc in the engagement concave portions 26, 28 is set so that the nut members 5, 6 can maintain a predetermined strength.

In order to protect the human body tissue, in the nut members 5, 6, the corners of rear ends 5a, 6a in the screw-in direction and the corners of end portions of the engagement concave portions 26, 28 are chamfered.

The nut member 5 and nut member 6 are formed to have identical dimensions, except the thickness thereof.

Such nut members 5, 6 can be manufactured, for example, by forming a titanium cylinder, then forming female threaded portions 25, 27 by cutting a thread on the inner peripheral surface, forming the engagement concave portions 26, 28 by machining the outer peripheral surface, chamfering the predetermined zones, and then performing a predetermined surface treatment.

The above-described rod 2, screw member 3, connector member 4, nut members 5, 6 and fixing member 7 are formed from a corrosion-resistant material such as titanium, and a coating film is formed on the entire surface thereof by anodization.

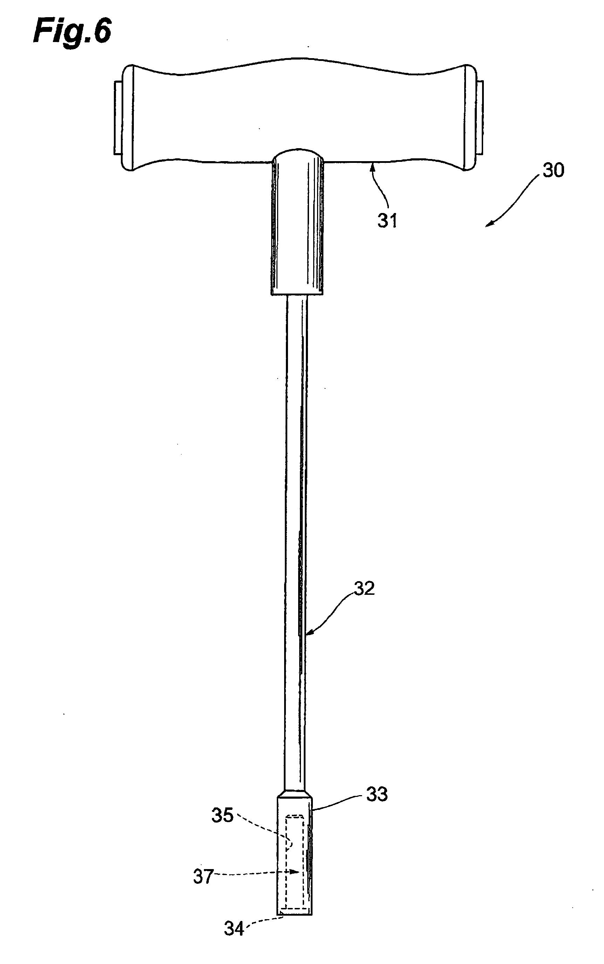

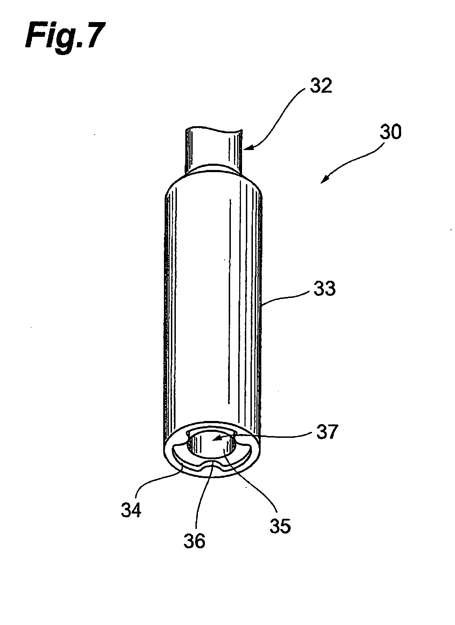

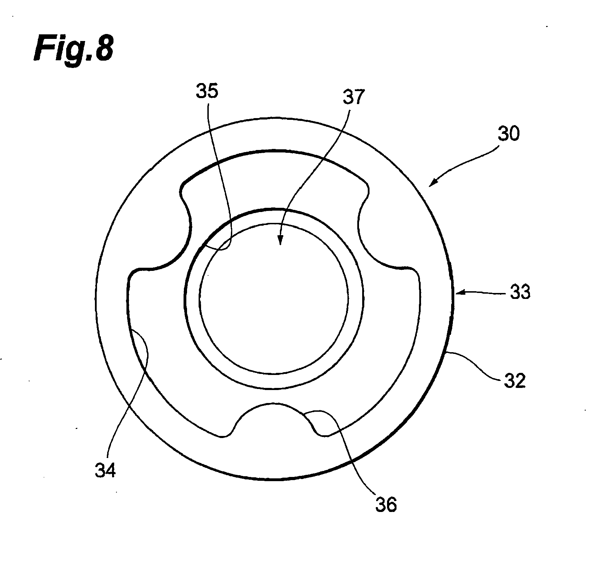

A nut driver of one embodiment of the present invention will be explained below. FIG. 6 is a front view illustrating the nut driver of the embodiment, FIG. 7 is a perspective view illustrating, with partial enlargement, the lower portion of the nut driver, and FIG. 8 is a bottom view of the nut driver.

As shown in FIG. 6 to FIG. 8, a nut driver 30 of the present embodiment is designed to rotate the nut members 5, 6 of the vertebra connection member 1 and comprises a T-shaped handle portion 31 and a shaft portion 32 that is detachably attached to the handle portion 31, held thereby so that it cannot rotate around the axis thereof, and extends linearly from the handle portion 31. A nut member holding portion 33 is provided at the distal end of the shaft portion 32.

The nut member holding portion 33 is formed to have a substantially cylindrical shape so as to hold the nut members 5, 6 that will be screwed onto the second male threaded portion 11 of the screw member 3, and a cavity portion 37 open at the distal end is formed in the inner peripheral surface of the nut member holding portion 33. A nut member insertion orifice 34 for inserting the nut members 5, 6 is formed in the distal end side (lower side in FIG. 6) of the cavity portion 37, and a male threaded portion insertion orifice 35 for inserting the second male threaded portion 11 of the screw member 3 is formed in the nut member insertion orifice 34 on the side of the handle portion 31 (upper side in FIG. 6).

The inner peripheral surface of the nut member insertion orifice 34 is formed to conform to the shape of the outer peripheral surface of the nut members 5, 6. More specifically, the inner peripheral surface of the nut member insertion orifice 34 is formed to have a diameter equal to, or slightly larger than the outer peripheral diameter of the nut members 5, 6, and an engagement convex portion 36 of a circular arc shape with a diameter equal to, or slightly less than that of the engagement concave portions 26, 28 is formed in a position corresponding to the engagement concave portions 26, 28 of the nut members 5, 6. The length (depth) of the nut member insertion orifice 34 is set to a length equal to that of the thinnest nut member 5, 6, or to a length less than that of the thinnest nut member 5, 6, so that the nut member other than the rotated nut member is nor rotated. The nut members 5, 6 and the nut member insertion orifice 34 may be set to any dimensions, provided that the nut member insertion orifice 34 is not engaged with the nut members 5, 6 or rotated in an idle mode when the nut member insertion orifice 34 is inserted onto the nut members 5, 6.

The inner peripheral surface of the male threaded portion insertion orifice 35 is formed to conform to the shape of the outer peripheral surface of the shaft portion 32. More specifically, the inner peripheral surface of the male threaded portion insertion orifice 35 is formed such that the diameter of the male threaded portion insertion orifice 35 is larger than the maximum diameter of the second male threaded portion 11, so that the second male threaded portion 11 can be freely inserted therein. The length (depth) of the male threaded portion insertion orifice 35 is set such that the second male threaded portion 11 does not reach the farthest end of the male threaded portion insertion orifice 35 when the nut member 5 is screwed on to the very end with the nut driver 30.

A method for connecting a plurality of vertebrae by using the vertebra connection member 1 and nut driver 30 of the present embodiment will be explained below.

First, the first male threaded portion 10 of the screw member 3 is screwed into a vertebra, the screw member insertion orifice 17 of the connector member 4 is inserted onto the second male threaded portion 11, and the position of the connector member 4 with respect to the screw member 3 is adjusted.

Where the position of the connector member 4 is determined, the nut member 5 is fitted into the nut member holding portion 33 so that the engagement convex portion 36 of the nut driver 30 mates with the engagement concave portion 26 of the nut member 5, and the nut member 5 is screwed onto the second male threaded portion 11 by rotating the nut driver 30. The second male threaded portion 11 that protrudes from the nut member 5 as the nut member 5 is screwed thereonto is accommodated within the male threaded portion insertion orifice 35.

Where the nut member 5 is screwed onto the second male threaded portion 11 and the screw member fixing portion 15 is fixed between the nut member 5 and the locking portion 12, the nut member 6 is also screwed, similarly to the nut member 5, onto the second male threaded portion 11 by using the nut driver 30 and the nut member 5 is prevented from playing.

Where other screw members 3 are likewise screwed into other vertebrae and the connector members 4 are fixed to the screw members 3, the rod 2 is inserted into rod insertion orifices 18 of each connector member 4, the fixing members 7 are screwed into the orifices 19 for the fixing member, and the rod 2 is fixed to the connector members 4. As a result, a plurality of vertebrae are connected by the vertebra connection members 1.

As described hereinabove, in the vertebra connection member 1 of the present embodiment, by forming substantially cylindrical nut members 5, 6 and forming engagement concave portions 26, 28 of a concave curved shape on the outer peripheral surfaces thereof, the nut members 5, 6 can be engaged with the nut driver 30 by the engagement concave portions 26, 28, without providing portions protruding from the substantially cylindrical outer peripheral surfaces. As a result, the nut member holding portion 33 of the nut driver 30 is not thin partially and the thickness of the nut member holding portion 33 can be thinned overall. Therefore, the space necessary for rotating the nut driver when the nut members 5, 6 are screwed onto the second male threaded portion 11 can be decreased, thereby making it possible to shorten the distance from the screw member insertion orifice 17 to the rod fixing portion 16 and to decrease the size of the connector member 4.

Further, with the vertebra connection member 1 of the present embodiment, because the engagement concave portions 26, 28 of the nut member 5, 6 that will be engaged with the nut driver 30 are formed to have a concave curved surface of a circular arc shape, the rotation force received by the nut members 5, 6 from the nut driver 30 can be received by the concave curved surface. As a result, the surface area of contact with the nut driver 30 is enlarged and damage of the nut members 5, 6 can be inhibited.

Further, with the vertebra connection member 1 of the present embodiment, because the engagement concave portions 26, 28 are formed to have a circular arc shape, the center thereof being in a point on a circumference forming an outer peripheral surface of the nut members 5, 6, the frontmost end in the rotation direction of engagement convex portions 3 6 that come into contact with the engagement concave portions 26, 28 is located on the inner side in the radial direction of the outer peripheral surface of the nut members 5, 6. As a result, the loss of rotation force received by the nut members 5, 6 from the nut driver 30 can be reduced and the nut members 5, 6 can be reliably rotated.

Further, with the vertebra connection member 1 of the present embodiment, because the engagement concave portions 26, 28 are formed with equal spacing in the circumferential direction of the nut members 5, 6, the rotation forces inputted from the engagement concave portions 26, 28 can be balanced, and the nut members 5, 6 can be smoothly rotated.

Where the engagement concave portion is formed in two locations or one location in the circumferential direction, the balance of forces inputted from the engagement concave portions can be easily lost. Therefore, the nut members are difficult to rotate smoothly. Conversely, where the engagement concave portions are formed in four or more locations in the circumferential direction, the processing cost of the engagement concave portions increases. By contrast, with the vertebra connection member 1 of the present embodiment, because the engagement concave portions 26, 28 are formed in three locations in the circumferential direction, the forces inputted from the engagement concave portions 26, 28 can be easily balanced and the nut members can be smoothly rotated, while inhibiting the processing cost.

With the nut driver 30 of the present embodiment, because the nut member holding portion 33 of the nut driver 30 is formed to have a substantially cylindrical shape so as to cover the entire circumference of the nut members 5, 6, the force applied outwardly and radially to the engagement convex portion 36 by the reaction from the engagement concave portions 26, 28 when the nut driver 30 is rotated is received by the nut member holding portion 33. Therefore, the loss of force rotating the nut members 5, 6 can be reduced and the nut members 5, 6 can be reliably rotated

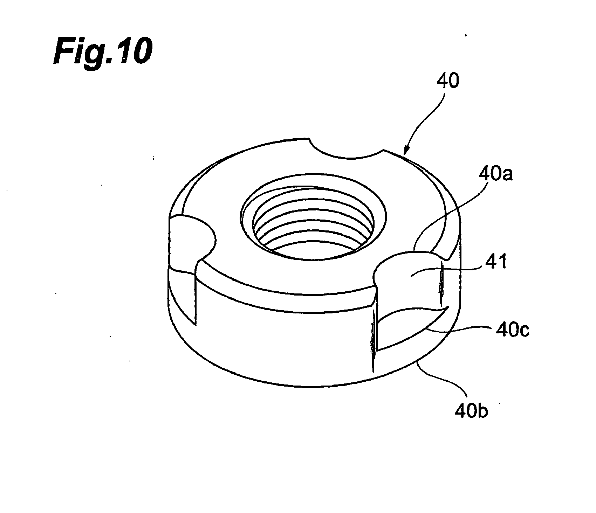

The present invention has been specifically explained hereinabove based on the embodiment thereof, but the present invention is not limited to the above-described embodiment. For example, in the above-described embodiment, the engagement concave portions 26, 28 are explained to be formed from the rear ends 5a, 6a in the screw-in direction of the nut members 5, 6 to the front ends 5b, 6b in the screw-in direction, but similar to the nut member 40 shown in FIG. 10 an engagement concave portion 41 may be also formed from a rear end 40a in the screw-in direction of the nut member 40 to a location 40c at a predetermined distance forward of a front end 40b in the screw-in direction. By thus providing a location where no engagement concave portion 41 is formed at the front end 40b in the screw-in direction, the nut driver 30 can be prevented from getting forward in the screw-in direction of the nut member 40. Therefore, the other nut member can be prevented from being erroneously rotated at the same time, and the screw-in position (or screw-in amount) of the nut member that is being presently screwed can be determined.

Further, in the above-described embodiment, the screw member insertion orifice 17 and rod insertion orifice 18 are explained as orifices that are formed to pass through the screw member fixing portion 15 and rod fixing portion 16, respectively, but they may be formed to have any shape, provided that the screw member 3 and rod 2 can be fixed. for example, one of those orifices may be formed to have an open U-like shape.

Further, in the above-described embodiment, all three engagement concave portions 26, 28 are explained to have identical shapes, but the shape thereof is not required to be the same, provided that they are formed to have a curved shape. Further, the number of engagement concave portions 26, 28 is not particularly limited and may be two or less and four or more.

Further, in the above-described embodiment, the second male threaded portion 11 is explained to be formed at the other end of the screw member 3. However, for example, a rod-like shape to be inserted into the nut members may be formed at the other end of the screw member 3, and the second male threaded portion may be formed in a position at a predetermined distance from the other end in the direction of the one end.

Further, in the above-described embodiment, the screw member 3 and connector member 4 are explained to be fixed by a double nut structure containing the nut member 5 and nut member 6. However, for example, with no use of the nut member 6, the screw member 3 and connector member 4 may be also fixed by a single nut structure containing only the nut member 5.

The explanation of the present invention presented hereinabove clearly demonstrates that various modifications of the present invention can be made. Such modifications should not deviate from the spirit and scope of the present invention, and all the improvements obvious to a person skilled in the art are included in the claims below.

## Claims

1. A vertebra connection member that connects a plurality of vertebrae via a rod, comprising
a screw member, in which a first male threaded portion to be screwed into a vertebra is formed at one end, a second male threaded portion is formed on a side closer to the other end than the first male threaded portion, and a locking portion having a larger diameter than the second male threaded portion is formed between the first male threaded portion and the second male threaded portion;
a connector member having formed therein a screw member insertion portion for inserting the screw member from a side closer to the second male threaded portion than the locking portion, thereby locking the locking portion, and a rod insertion portion into which the rod is inserted, the connector member connecting the screw member and the rod; and
a substantially cylindrical nut member having formed on an inner peripheral surface thereof a female threaded portion that conforms in shape to the second male threaded portion and on an outer peripheral surface thereof one and more engagement concave portions of a concave curved shape oriented forward in a screw-in direction from a rear end in the screw-in direction of the female threaded portion.

2. The vertebra connection member according to claim 1, wherein the engagement concave portions are formed to have a circular arc shape, the center thereof being in a point on a circumference forming an outer peripheral surface of the nut member.

3. The vertebra connection member according to claim 1, wherein the engagement concave portions are formed up to a front end in the screw-in direction.

4. The vertebra connection member according to claim 1, wherein the engagement concave portions are formed, in the screw-in direction of the female threaded portion, at the front end up to a point where a predetermined distance begins.

5. The vertebra connection member according to claim 1, wherein the engagement concave portions are formed with equal spacing in a circumferential direction.

6. The vertebra connection member according to claim 1, wherein the engagement concave portions are formed in three locations.

7. A nut driver comprising a nut member holding portion that covers an outer circumferential surface of the nut member according to claim 1, wherein
the nut member holding portion has an engagement convex portion in a form of a convex curved surface that engages with the at least one engagement concave portions.
